# EUROPEAN PATENT SPECIFICATION

(11) **EP 1 368 045 B1**
(45) Date of publication and mention of the grant of the patent: **19.07.2006**
(21) Application number: 02702184.9
(22) Date of filing: 12.03.2002
(51) Int. Cl.: A61K 36/87, C12F 3/06, A61P 9/12

(54) **PROCESS FOR OBTENTION OF DECOCTIONS OF VITIS LABRUSCA AND VITIS VINIFERA SKINS**
VERFAHREN ZUR GEWINNUNG VON DEKOKTEN AUS SCHALEN VON VITIS LABRUSCA UND VITIS VINIFERA
PROCEDE DE PREPARATION DE DECOCTIONS DE PEAUX DE VITIS LABRUSCA ET DE VITIS VINIFERA

(30) Priority: 13.03.2001 BR 0106382
(43) Date of publication of application: 10.12.2003
(73) Proprietor: Universidade Do Estado Do Rio De Janeiro, CEP-20551-030 Rio de Janeiro (BR)
(72) Inventor: MOURA, R. S., Uni. Do Estado Do Rio De Janeiro, CEP 20551-030 Rio de Janeiro (BR)
(74) Representative: Pelayo de Sousa Henriques, Rui
(86) International application number: PCT/BR2002/000038
(87) International publication number: WO 2002/072118

(56) References cited:
- EP-A2- 0 275 224
- DE-A- 2 129 654
- FR-A1- 2 775 686

## Description

Process for obtention of decoctions of *Vitis labrusca* and *Vitis vinifera* skins; Process for obtention of hydro-alcoholico and hydro-alcoholic ethyl acetate from the decoctions; pharmaceutical preparations containing the decoction and the extracts and therapeutic indications of the preparations in the prevention and treatment of arterial hypertension and other cardiovascular diseases.

### I) Field of Invention

The present invention deal with products that have anti-hypertensive properties, process to obtain products from plants belonging to *Vitacea* family, more specifically to *Vitis labrusca* and *Vitis vinifera* species, application of those products, process to obtain those products, more specifically a process to obtain a decoction from skins of those plants, more specifically a process to obtain an of hydro-alcoholic and hydro-alcoholic- ethyl acetate extracts from those decoctions, more specifically, a process to obtain pharmaceutical preparations containing these products and therapeutic indications of pharmaceutical preparations in the treatment of arterial hypertension and diseases caused by arterial hypertension.

### II) Invention Antecedents

Arterial hypertension is a disease with high prevalence among adult population and induces many deleterious effects in hypertensive patients, including cardiac, kidney and cerebral dysfunction's. Arterial hypertension is at the moment one of the largest causes of death. Therefore, pharmacological treatment, that have the scope to reduces the high level of arterial blood pressure and the cardiovascular complications from arterial hypertension, is helpful for the patient and supported by health public agency. Usually the pharmacological treatment of hypertension is obtained with use of diuretics, beta blocking agents, inhibitors of the renin-angiotensin system, inhibitors of the sympathetic system and vasodilators compounds.

Data from the literature suggest that cardiovascular mortality be inversely related to moderate ingestion of alcoholic beverage. Leger et al., L: Lancet 1:1017-1020, 1979 suggested that daily ingestion of moderate amount of red wine may be responsible for the low incidence of coronary heart disease. This observation was coined as "French Paradox" (Lancet 338:464-486, 1991) since the incidence of coronary heart disease in the some part of France, where the ingestion of a high fat diet is not correlate with a high incidence of coronary heart disease, as observed in other countries. One explanation for the French Paradox could be related to the presence of polyphenols in the red wine, compounds that could acts on the metabolism of LDE, an important risk factor for coronary heart disease.

At the moment the mechanisms of the protection induced by small intake of wine on the reduction on cardiac mortality is not know, but probably an action on the metabolism of lipids may be taken in consideration. However considered that arterial hypertension is an important risk factors for coronary heart disease, the present invention suggest a protective mechanism based on the experimental data object of this patent, that showed that products obtained from grape skins reduce the development of arterial hypertension in rats, and also reduce the high levels of arterial blood pressure in experimental hypertension.

### III) Summary of the Invention.

The present invention refers to a process to obtain products that have anti-hypertensive properties. Refer also of those products that are obtained from plants belonging to *Vitacea* family, more specifically *Vitis vinifera* and *Vitis labrusca* species, in particularly, to a process to obtain products that include extraction of those products from the fruits of those plants.
More particularly, the present invention, refer the to a process to obtain products that have anti-hypertensive activity, that include separation of skins, pulps and seeds of the fruits, to obtain a decoction from the skins of the fruits and extraction of the decoction with solvents, particularly solvents physiologically acceptable as ethanol, ethyl acetate and/or its mixtures, and posterior process of the decoctions and extracts to obtain products with pharmacological activities.
More particularly, refer also the present invention, methods to obtain products with anti-hypertensive properties as a decoction, that include an extraction with a solvent physiologically acceptable, as for instance, water as a decoction for 3 to 30 minutes.
Also, refer the present invention to process the decoctions and extracts obtain products with pharmacological activities.
Also, refer the present invention process to obtain products as hydro-alcoholic and hydro-alcoholic- ethyl acetate extracts, and posterior separation, concentration and lyophilization to obtain the products.

Also, the present invention refer, as mention above, to the before mentioned products per se and also the utilization of the before mentioned products and medicines containing the before mentioned products, with ant-hypertensive properties.

### IV) Condensed description of the methodology used.

The present invention is supported by scientific data obtained in chemical and pharmacological experimentations. Below we give a brief view of the investigative process that supports the invention.

### IV.A) Method to obtain the grape-skin decoctions.

*Vitis labrusca* and *Vitis vinifera* fruits were washed and after separation from the pulps, the skins were put inside a recipient made of neutral glass or stain-less steel, containing a certain amount of distilled water, boiled, minced and left macerated for a certain period of time, and further filtered in order to obtain the liquid phase of the decoction. The liquid phase is concentrated in a low-pressure rotator evaporator at approximately 40° C and then lyophilized. The lyophilized is kept frozen (-20°C).

### IV.B) Method to obtain the grape-skin hydro-alcoholic extract from the skin-decoction.

The fruits of *Vitis labrusca* or *Vitis vinifera* were washed and after separation from the pulps, the skins were put inside recipient made of neutral glass or stainless-steel containing a certain amount of destined water and boiled. The decoction was minced, extract with ethanol, same amount of water e then left macerated for a certain period of time. The mixture is filtered, the liquid phase is kept refrigerated and the semi-solid phase can be again extract with a mixture of water/ethanol (v: v) for one or two time. The liquid phases are concentrated in a low-pressure rotator evaporator at approximately 40°C and then lyophilized and kept at -20°C.

### IV.C) Method to obtain grape-skin hydro-alcoholic-ethyl acetate extract from the skin-decoction.

The fruits of *Vitis labrusca* or *Vitis vinifera* were washed and after separation from the pulps, the skins were put inside recipient made of neutral glass or stainless-steel containing a certain amount of destined water, boiled, minced, extract with a mixture of ethanol- ethyl acetate -decoction (v:v:v) and then left macerated for a certain period of time. The mixture is filtered, the liquid phase is kept refrigerated and the semi-solid phase can be again extract with a mixture of water/ethanol/acetate ethyl (v:v:v) for one or two time, and the liquid phases are concentrated in a low pressure rotator evaporator at approximately 40° C and then lyophilized and kept at -20° C.

### IV.D) Pharmacodynamic and pharmacotechnical methods.

The pharmacological activities of the products obtained from *Vitis labrusca* and *Vitis vinifera*-skins were assessed by pharmacodynamical tests that assessed the anti-hypertensive action of the products in spontaneous hypertensive rats, Doca-salt hypertensive rats and L-NAME hypertensive rats. The pharmacotechnical method refers the way to obtain capsules containing the lyophilized of the extracts obtained from *Vitis labrusca* and *Vitis vinifera* skins.

### V.) Detailed description of the Invention.

In the ambit of the present invention, the fruits of *Vitis labrusca* and *Vitis vinifera* species, before been submitted to the process of extraction, according to the invention, if not utilized after the harvest, can be stored for long periods at the temperature from +4 to - 20° C.

### V.A) Method to obtain the grape-skin decoction.

Fruits of *Vitis labrusca* or *Vitis vinifera* are washed in water, and the skin is separated from the pulp. The skins are washed in water e later put inside of neutral glass or stainless steel recipient in the proportion of 1 to 100 g of skin to 100 ml of water, for instance 25 g /100 ml and boiled for 3 to 60 minutes, for instance about 5 minutes. After the time of boiling, the decoction is left to cool to 20 to 90° C, for instance 75° C and minced. The minced decoction was macerated for a certain period of time (1 hour to 30 days) for instance 6 hours inside a refrigerate at 4° C or at room temperature for instance 25° C under agitation. The decoction is filtered in a sieve with 0.1 to 1.0-mm pore, for instance 0.2 mm, being also filtered through gauze and finally filtered through a paper filter Whartman n.l. The liquid phase is concentrated in a low-pressure evaporator at a temperature of 30 to 60°C for instance 40 °C and then lyophilized and kept under -4 to - 70° C.

### V.B) Method to obtain the grape-skin hydro-alcoholic extract from the decoction.

The grape-skin decoction, after been minced short after boiling, is extracted with ethanol 95% in a proportion of decoction/ethanol (v:v) of 1:0.5 to 1:10, for instance 1:1. That mixture is minced and macerated for a certain period of time of 3 hours to 30 days, for instance 6 hours and kept inside a refrigerator at 4° C or at room temperature at 25°C and shaken. At the end of the maceration period it is filtered through a sieve with 0.1 to 1 mm pores, for instance 0.2 mm, being also filtered through gauze and finally filtered through a filter paper, Whartman n.l. The semi-solid phase can be extracted again in the same conditions as described above for I or 3 times, for instance 2 times. The liquid phase of the first extraction is kept inside a refrigerator at 4° C and then added to the liquid phase of the other extractions. The final liquid phase is concentrated under low pressure evaporator at a temperature of 35 to 65°C, for instance 40°C e then lyophilized and kept at -4 °C to -70° C, for instance -20° C.

### V.C) Method to obtain the grape-skin hydro-alcoholic-acetate of ethyl extract from the decoction.

The decoction, after been minced after boiling, is extracted with a mixture of decoction/ethanol/ethyl acetate (v:v:v) of variable proportion for instance 1:1:1. That mixture is minced and macerated for a certain period of time of 3 hours to 30 days, for instance 6 hours and kept inside a refrigerator at 4° C or at room temperature at 25° C and shaken. At the end of the maceration period the extract is filtered through a filter paper Whartman n, I and the liquid phase kept inside a refrigerator at 4° C and the semi-solid phase can be again extracted at the same condition as described above for one or three times, for instance two times. The liquid phase of the first extraction is kept inside a refrigerator at 4° C and then added to the liquid phase of the other extractions. The final liquid phase is concentrated under low pressure evaporator at a temperature of 35 to 65° C, for instance 40° C e then lyophilized and kept at 4 C to -70° C, for instance -20° C.

### VI.) General Observation.

The specific procedure described in item V.A to V.C above, were described not with the scope to limit, but wit the scope to illustrate the possibilities of realization of the present invention, with ambit is limited only by the claim annex.

### VII.) Pharmacodynamical and Pharmacotechnical Methods

The pharmacological activities of the various products obtained from the *Vitis labrusca* and *Vitis vinifera* skins were assessed by pharmacodynamic methods that study the anti-hypertensive activity of the products in the following models of experimental hypertension: DOCA-salt hypertension, spontaneous hypertensive rats (SHR) and hypertension induced by inhibition of nitric oxide synthase. The pharmacothechnical method refer to the method to obtain capsules containing the lyophilized residue of the products obtained from *Vitis labrusca* and *Vitis vinifera* skins.

### VIII) Illustrative example of the Invention

Below are examples with the objective to illustrate and not to limit the present invention, which purpose, as mention above, has its delimitation's in the claim annex.

### VIII.A) Method to obtain the lyophilized of the Vitis labrusca grape-skin decoction

Approximately 2 000 g of *Vitis labrusca* fruits were washed in tap water. The skins were isolated from the pulps and washed in tap water during approximately 3 minutes. The skin (1000g) were boiled in 4000 mL of distilled water for 5 minutes. After boiling, while the decoction was still warm, the decoction was minced at approximately 80°C and left for maceration for 6 hours under shaking. The decoction was filtered through a sieve with 0.2 mm pores and then filtered in gauze and then through a filter paper Whatman n.1. The liquid phase, obtained after filtration was concentrated under low pressure evaporator at 45° C, lyophilized and kept at -20° C, until the day of use.

### VIII.B) Method to obtain the lyophilized of the Vitis vinifera grape-skin decoction

Approximately 2 000 g of *Vitis vinifera* fruits were washed in tap water. The skins were isolated from the pulps and washed in tap water during approximately 3 minutes. The skin (1000g) were boiled in 4000 mL of distilled water for 5 minutes. After boiling, while the decoction was still warm, the decoction was minced at approximately 80°C and left for maceration for 6 hours under shaking. The decoction was filtered through a sieve with 0.2 mm pores and then filtered in gauze and then through a filter paper Whatman n.l. The liquid phase, obtained after filtration was is concentrated under low-pressure evaporator at 45° C, lyophilized and kept at -20° C, until the day of use.

### VIII.C) Method to obtain the lyophilized of the hydro-alcoholic extract obtained from Vitis labrusca grape-skin decotion.

Approximately 1 000 g of *Vitis labrusca* fruits were washed in tap water. The skins were isolated from the pulps and washed in tap water during approximately 3 minutes. The skin (500g) were boiled in 2 L of distilled water for 5 minutes. After boiling, the decoction was minced. Two liter of ethanol was added to the decoction and minced. The extracted was left macerating for 6 hours and filtered through a sieve, pores 0.2-mm and then filtered through a filter paper Whartman n.l. The liquid phase, obtained after filtration was is concentrated under low-pressure evaporator at 45° C, to evaporate the ethanol and then lyophilized to obtain the lyophilized of the hydro-alcoholic extract of the *Vitis labrusca* decoction.

### VIII.D) Method to obtain the lyophilized of the hydro-alcoholic extract obtained from Vitis vinifera grape-skin decoction.

Approximately 1 000 g of *Vitis vinifera* fruits were washed in tap water. The skins were isolated from the pulps and washed in tap water during approximately 3 minutes. The skin (500g) were boiled in 2 L of distilled water for 5 minutes. After boiling, the decoction was minced. Two liters of ethanol was added to the decoction and minced. The extracted was left macerating for 6 hours and filtered through a sieve, pores 0.2-mm and then filtered in a filter paper Whartman n.l. The liquid phase, obtained after filtration was is concentrated under low-pressure evaporator at 45° C, to evaporate the ethanol and then lyophilized to obtain the lyophilized of the hydro-alcoholic extract of the *Vitis vinifera* decoction.

### VIII.E) Method to obtain the lyophilized of the hydro-alcoholic- ethyl acetate extract obtained from Vitis labrusca grape-skin decoction.

Approximately 1 000 g of *Vitis labrusca* fruits were washed in tap water. The skin (500g) were isolated from the pulps and washed in tap water during approximately 3 minutes. The skins were boiled in 2 L of distilled water for 5 minutes. After boiling, the decoction was minced. One liter of ethanol plus one liter of ethyl acetate were added to the decoction and minced. The extracted was left macerating for 6 hours and filtered through a sieve, pores 0.2-mm and then filtered in a filter paper Whartman n.l. The liquid phase, obtained after filtration was concentrated under low-pressure evaporator at 45° C, to evaporate the ethanol and then lyophilized to obtain the lyophilized of the hydro-alcoholic-extract of the *Vitis labrusca* decoction.

### VIII.F) Method to obtain the lyophilized of the hydro-alcoholic-ethyl acetate extract obtained from Vitis vinifera grape-skin decoction.

Approximately 1 000 g of *Vitis virtifera* fruits were washed in tap water. The skins were isolated from the pulps and washed in tap water during approximately 3 minutes. The skins were boiled in 2 L of distilled water for 5 minutes. After boiling, the decoction was minced. One liter of ethanol plus one liter of ethyl acetate were added to the decoction and minced. The extracted was left macerating for 6 hours and filtered through a sieve, pores 0.2-mm and then filtered in a filter paper Whartman n.l. The liquid phase, obtained after filtration was concentrated under low-pressure evaporator at 45° C, to evaporate the ethanol and then lyophilized to obtain the lyophilized of the hydro-alcoholic extract of the *Vitis vinifera* decoction.

### IX). Example of biological tests performed with the products of the invention.

The anti-hypertensive activity of lyophilized from various products was access by testing its efficacy of the lyophilized to reduce the levels of experimental arterial hypertension and to reduce the development of hypertension in rats. The anti-hypertensive activity was accessed in adult male Wistar rats, spontaneous hypertesive or made hypertensive by the following methods: nitric oxide inhibition by use of an analogue of L-arginine, that is, L-NAME, and subcutaneous injection of DOCA followed by orally administration of saline in uninephrectomized rats; Arterial blood pressure was measured in the tail of rats by a noninvasive method while the rats were awake, using a cuff and a sensor connected to equipment manufactured by Letica-Barcelona-Spain. The lyophilized was administrated orally, in the drinking water, so that the rats were treated with the products continuously during the period of treatment. Arterial pressure was measured three times per week before and during the treatment with the lyophilized. The values of arterial blood pressure were compared using Student's test and the differences were considered significantly when p < 0,05.

### IX.A). Effects of the lyophilized obtained from the decoction of Vitis labrusca skins in the arterial hypertension induced by inhibition of nitric oxide synthesis in rats.

Adult, male Wistar rats (250 -350 g, n = 10) were kept cage with no more than 4 animals por cage. Mean arterial blood pressure was measured noninvasively, by Lctica (Barcelona) equipment. A cuff and a sensor around the tail of the animal were connected to the equipment. The cuff is inflated automatically in order the measure the mean arterial blood pressure.
After a period of adaptation the experimental conditions, for measurement the mean arterial blood pressure, the pharmacodynamical test was started. During the adaptation period, body weight was estimated three times a week, the animals received tap water and food at libitum, and the daily intake of water was estimated.

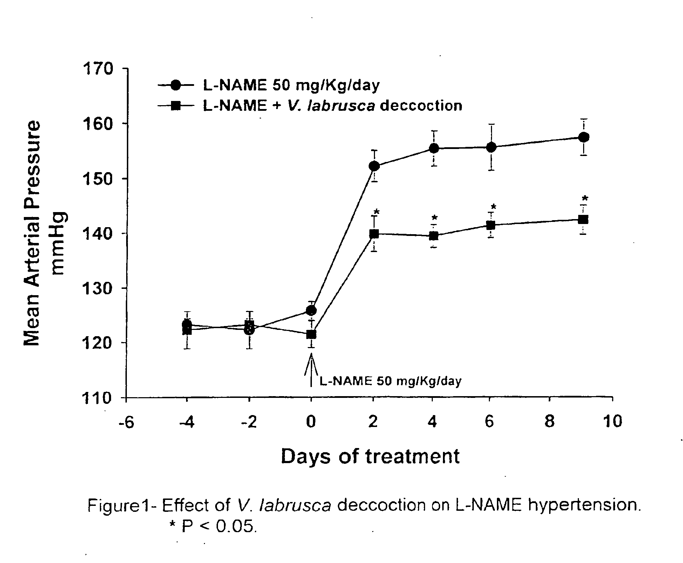

The animals were divided in two groups of 5 rats. One group (control) was treated orally with L-NAME, 50 mg/kg/day, diluted in the drinking water. The other group was also treated with L-NAME, 50 mg/kg/day plus 100 mg/kg/day of the lyophilized of the decoction of *Vitis labrusca* in the drinking water. Figure 1 show the anti-hypertensive effect of the lyophilized of the decoction of Vitis labrusca in this particular experiment.

### IX.B) Effects of the lyophilized of the hydro-alcoholic extract obtained from the decoction of Vitis labrusca skins in the arterial hypertension induced by inhibition of nitric oxide synthesis in rats.

Adult, male Wistar rats (250 -350 g, n = 12) were kept cage with no more than 4 animals por cage. Mean arterial blood pressure was measured noninvasively, by Letica (Barcelona) equipment. A cuff and a sensor around the tail of the animal were connected to the equipment. The cuff is inflated automatically in order the measure the mean arterial blood pressure. After a period of adaptation the experimental conditions, for measurement the mean arterial blood pressure, the pharmacodinamical test was started. During the adaptation period, body weight was estimated three times a week, the animals received tap water and food at libitum, and the daily intake of water was estimated.

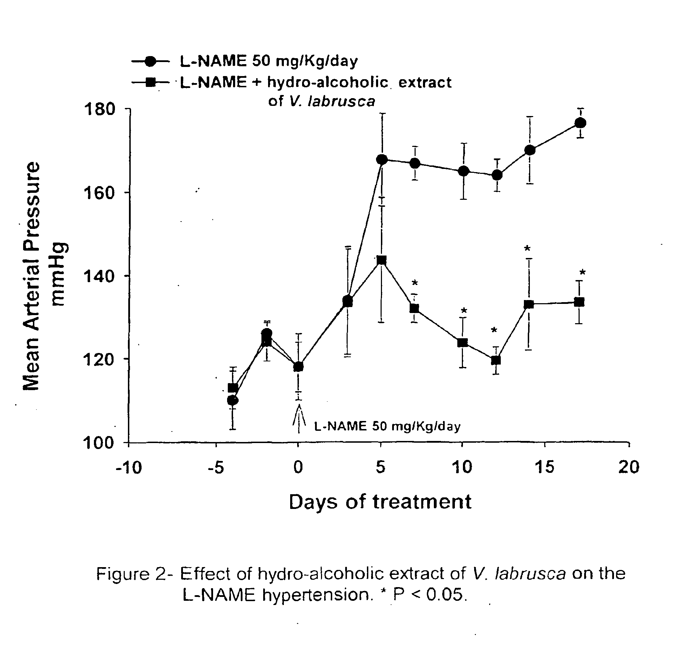

The animals were divided in two groups of 6 rats. One group (control) was treated orally with L-NAME, 50 mg/kg/day, diluted in the drinking water. The other group was also treated with L-NAME, 50 mg/kg/day plus 100 mg/kg/day of the lyophilized of the hydro-alcoholic extract of decoction of *Vitis labrusca* in the drinking water, five days after the beginning of treatment with L-NAME. Figure 2 show the anti-hypertensive effect of the lyophilized of the hydro-alcoholic extract obtained from the decoction of *Vitis labrusca* in this particular experiment.

### IX.C) Effects of the lyophilized of the hydro-alcoholic-acetate of ethyl extract obtained from the decoction of Vitis labrusca skins in the arterial hypertension induced by inhibition of nitric oxide synthesis in rats.

Adult, male Wistar rats (250 -350 g, n = 12) were kept cage with no more than 4 animals por cage. Mean arterial blood pressure was measured noninvasively, by Letica (Barcelona) equipment. A cuff and a sensor around the tail of the animal were connected to the equipment. The cuff is inflated automatically in order the measure the mean arterial blood pressure.

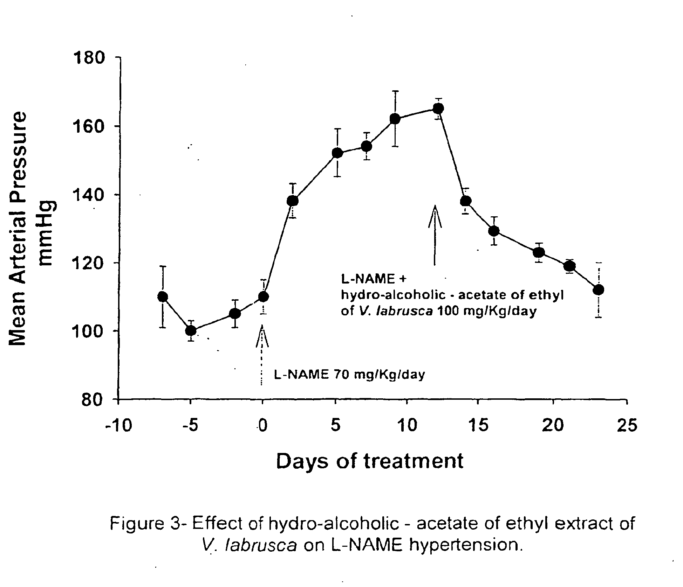

During the adaptation period, the animal received food and water "as libitum" and the daily intake of water was estimated. Body weight was estimated three times a week. After the levels of basal pressure were obtained, the rats were treated with L-NAME 70 mg/kg/day in the drinking water. Once the arterial pressure reached elevated level, the animal was treated with 100 mg/kg/day of the lyophilized of the hydro-alcoholic- ethyl acetate plus L-NAME. As can been observed in figure 3, the extract induced a significant anti-hypertensive effect in this particular experiment.

### IX.D) Effects of the lyophilized of the hydro-alcoholic-ethyl acetate extract obtained from the decoction of Vitis labrusca skins in rats with spontaneous hypertension.

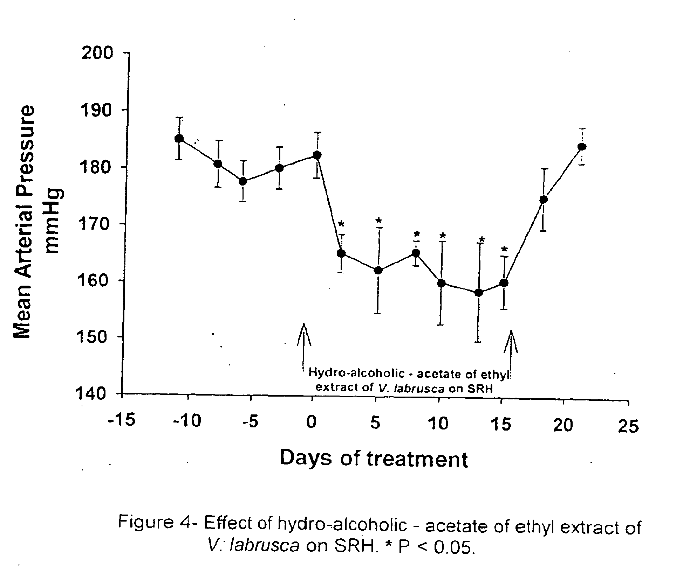

The antihypertensive activity of hydro-alcoholic-ethyl acetate extract obtained from the decoction of *Vitis Labrusca* was accessed in five spontaneous hypertensive rats weighting 250 to 350 g. Before the beginning of the treatment with the extract the arterial pressure was measured during two weeks, when after that period, the animals were treated orally with lyophilized of the extract 100 mg/kg/day. As showed in figure 4, oral treatment with the extract induced a significant anti-hypertensive response in this particular kind of experimental hypertension.

### IX.E) Effects of the lyophilized of the hydro-alcoholic extract obtained from the decoction of Vitis labrusca skins in rats with DOCA-Salt hypertension.

Twelve Wistar male rats, weighting 250 to 350 g were divided in two groups, each group kept in different cages. All rats were were uninephrectomized. Arterial pressure was measured non invasively using a Letica (Barcelona) equipment, that include a cuff, a sensor, put around the rat tail and connected to the equipment that inflate automatically the cuff and therefore measure the level of mean arterial pressure.

Seven days after the nephrectomy, the animal is adapted to the pressure measurement procedure and received food and water "ad libitun" and body weight was estimated three times a week.

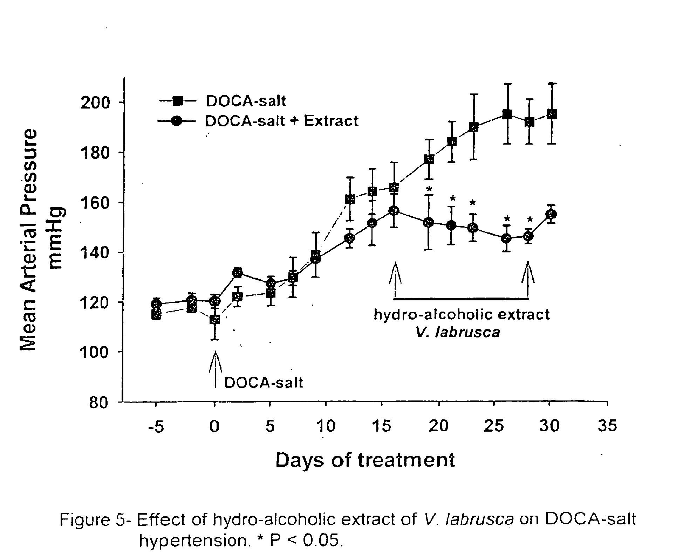

After the period of adaptation, the animals were treated subcutaneously with 25 mg/kg/week and received saline as drinking water. Arterial pressure was measured three times a week. After the beginning of the treatment, the arterial blood pressure started to elevate. Sixteen days after the beginning of treatment, when the arterial blood pressure had reached a high level, 6 rats were treated orally with the extract 100 mg/kg/day. The other group of 6 rats were treated with only DOCA plus saline. As showed in figure 5, the extract induced a significant reduction of the mean arterial blood pressure in hypertensive rats.

### IX.F) Effects of the lyophilized obtained from the decoction of Vitis vinifera skins in the arterial hypertension induced by inhibition of nitric oxide synthesis in rats.

Adult, male Wistar rats (250 - 350 g, n = 11) were kept cage with no more than 4 animals por cage. Mean arterial blood pressure was measured noninvasively, by Letica (Barcelona) equipment. A cuff and a sensor placed around the tail of the animal were connected to the equipment. The cuff is inflated automatically in order the measure the mean arterial blood pressure.

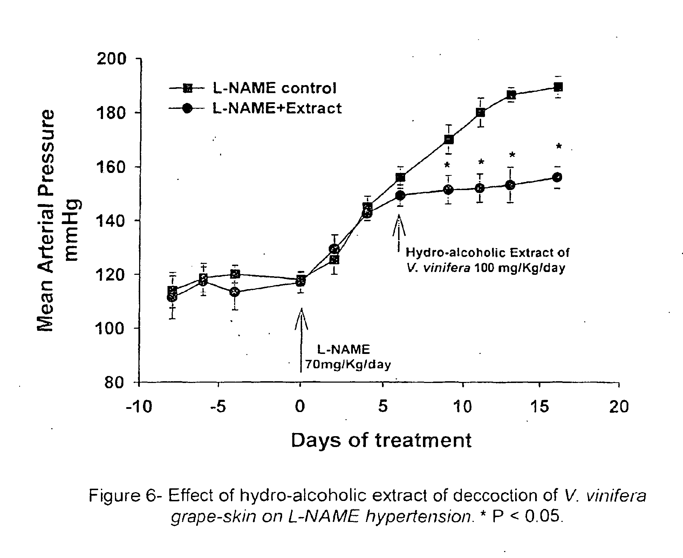

The animals were submitted to a period of adaptation of the experimental conditions, for measurement the mean arterial blood pressure. During the adaptation period, the animals received tap water and food "ad libitum", and the daily intake of water was estimated. Body weight was estimated three times a week.
After the basal pressure levels are obtained, one group (control, n = 5) was treated orally with 70 mg/kg/day L-NAME in drinking water. The other group (n = 6) slx days after beginning L-NAME treatment, was treated with 100 mg/kg/day of lyophilized hydro-alcoholic extract obtained from the decoction of Vitis vinifera skin plus L-NAME in the drinking water. As shown in figure 6, the extract induced a significant anti-hypertensive effect in this particular experiment.

### IX.G) Effects of the lyophilized of the hydro-alcoholic-ethyl acetate extract obtained from the decoction of Vitis vinifera skins in the arterial hypertension induced by inhibition of nitric oxide synthesis in rats.

Adult, male Wistar rats (250 -350 g, n = 11) were kept cage with no more than 4 animals por cage. Mean arterial blood pressure was measured noninvasively, by Letica (Barcelona) equipment. A cuff and a sensor placed around the tail of the animal were connected to the equipment. The cuff is inflated automatically in order the measure the mean arterial blood pressure.

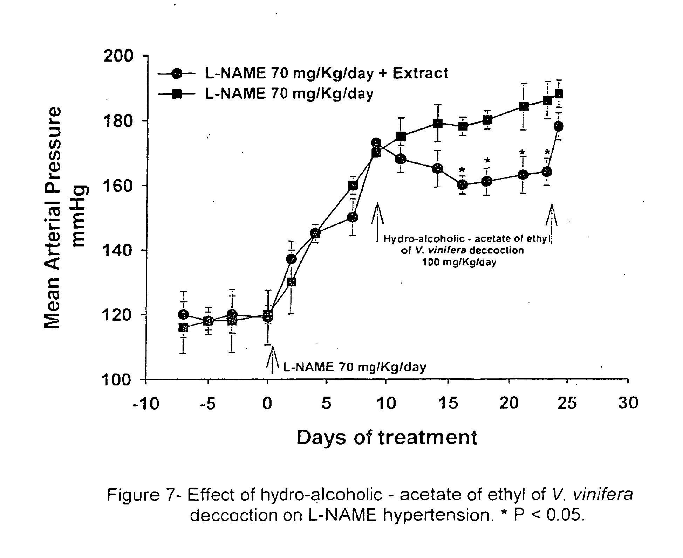

The animals were divided in two groups, were submitted to a period of adaptation of the experimental conditions, for measurement the mean arterial blood pressure. During the adaptation period, the animals received tap water and food "ad libitum", and the daily intake of water was estimated. Body weight was estimated three times a week. After the basal arterial pressure levels are obtained, both groups were treated orally with 70 mg/kg/day L-NAME in drinking water. After the arterial blood pressure reached high levels, due to L-NAME treatment, one group (n = 5) received only L-NAME and the other group (n = 6) was treated with L-NAME plus the extract, 100 mg/kg/day orally. This treatment induced a significant reduction of the high levels of arterial pressure, when compared with the control group, as showed in figure 7.

### X) Pharmacotechnical aspects of the preparation of capsules and tablets containing the dry residue of the various fractions obtained from skin of the fruits of Vitis labrusca and Vitisvinifera.

The capsules and/or tablets containing 100 to 500 mg of lyophilized of Vitis labrusca or Vitis vinifera were obtained according to the usual pharmacotechnical procedures. The capsules were obtained in order to contain 100 to 500 mg, for instance 250 mg of the lyophilized plus cornstarch and colloidal silicon dioxide. Each capsule could have the following composition:

| | | |
|---|---|---|
| Lyophilized | 250 mg | 55.5% |
| Corn starch | 200 mg | 44.4% |
| Colloidal silicon dioxide | 0.5 mg | 0.1% |
| Total | 450.5 mg | 100% |

Cornstarch was added to complete the total mass of the capsule to approximately 450 mg. Colloidal silicon dioxide was used to adsorb humidity and to facilitate the preparation of the capsules.

## Claims

1. Process to obtain decoction of skins of *Vitis labrusca* or *Vitis vinifera*, **characterized by** the following steps:
a) Separate the fruit into skins and pulps;
b) Wash the skin obtained in step (a) in tap water for 3 minutes;
c) Submit the skins of step (b) to a process of extraction in boiling water for 3 to 10 minutes to obtain the decoction;
d) Mince the decoction of step (c) with a mince during 3 to 5 minutes and macerate during 6 hours to 10 days at room temperature or at 4°C;
c) Filter the macerate obtained in step (d) in a sieve with 0.2 to 1 mm pores and subsequently in a filter paper;
f) Concentrate the liquid phase obtained in step (e) in a rotator evaporator at low pressure and at a temperature of 35 to 50 °C;
g) Lyophilize the concentrated liquid phase in order to obtain a lyophilized of the decoction with pharmacological activities;

2. . Process according to claim 1 **characterized by** the reason that the extraction of step (c) takes 5 minutes.

3. Process according to claim 1 **characterized by** the reason that the time of maceration is 24 hours.

4. Process, according to claim 1 **characterized by** the reason that the concentration of the liquid phase (step f) is obtained at 40°C.

5. Process to obtain a hydro-alcoholic extract **characterized by** the reason that in addition the decoction obtained in step (c) of claim 1 is extracted with the solvent ethanol in the proportion of 1:1, v:v and minced strongly during 3 to 5 minutes and then macerated for 6 hours to 10 days at room temperature or at 4°C; therefore obtaining the hydro-alcoholic extract of the decoction.

6. Process according to claim 5 **characterized by** the reason that the macerate is filtered throughout a sieve with 0.2 to 1.0-mm pores and subsequently in filter paper.

7. Process according to claim 6 **characterized by** the reason that the liquid phase is concentrated in a rotator evaporator at low pressure and at 35 to 50° C.

8. Process according to claim 7 **characterized by** the reason that the liquid phase after concentration is lyophilized to obtain the lyophilized hydro-alcoholic extract of the decoction with pharmacodynamical activity.

9. Process to obtain a hydro-alcoholic-acetate of ethyl extract **characterized by** the reason that in addition the decoction obtained in step (c) of claim 1 is extracted with the solvents ethanol and acetate of ethyl in the proportion of 1:1:1, v;v;v and minced strongly during 3 to 5 minutes and then macerated for a period of 6 hours to 10 days at room temperature or at 4°C to obtain the hydro-alcoholic-acetate of ethyl extract of the decoction.

10. Process according to claim 9 **characterized by** the reason that the macerate obtained is filtered in sieve with 0.2 to 1.0 mm pores and then in filter paper.

11. Process according to claim 10 **characterized by** the reason that the liquid phase is concentrated in rotator evaporator under low pressure and at 35 to 50 °C.

12. Process according to claim 11 **characterized by** the reason that the liquid phase after being concentrated is lyophilized to obtain the lyophilized of the hydro-alcoholic-acetate of ethyl extract of the decoction with pharmacodynamical activity.

13. Pharmaceutical preparations **characterized by** the reason that they contain 200 to 500 mg of lyophilized of the decoction of *Vitis labrusca* or *Vitis vinifera* of claims 1 to 4 and in addition 200 mg of corn amide and 0.5 mg of colloidal silicon dioxide.

14. Pharmaceutical preparations **characterized by** the reason that they contain 200 to 500 mg of lyophilized of the hydro-alcoholic extract obtained from the decoction of *Vitis labrusca* or *Vilis vinifera* of claims 5 to 8 and in addition 200 mg of corn amide and 0.5 mg of colloidal silicon dioxide.

15. Pharmaceutical preparations **characterized by** the reason that they contain 200 to 500 mg of lyophilized of the hydro-alcoholic-acetate of ethyl extract obtained from the decoction of *Vitis labrusca* or *Vitis vinifera* of claims 9 to 12 and in addition 200 mg of corn amide and 0.5 mg of colloidal silicon dioxide.

16. Use of the pharmaceutical preparations pointed in claims 13, 14 and 15 for the manufacture of a medicament for the prevention and treatment of arterial hypertension and diseases induced by arterial hypertension.

17. Use according to claim 16, where the medicament is for human and veterinary applications.

## Patentansprüche

1. Das Verfahren für den Erhalt von Schalenabkochungen von *Vitis labrusca* oder *Vitis vinifera* charakterisiert sich durch folgende Schritte:
a) die Früchte in Schalen und Fruchtfleisch sortieren:
b) die im Schritt (a) erhaltenen Schalen 3 Minuten lang in laufendem Wasser waschen;
c) die im Schritt (b) erhaltenen Schalen 3 bis 10 Minuten lang in kochendem Wasser für ein Extraktionsprozess für den Erhalt der Abkochung aussetzen;
d) die im Schritt (c) erhaltene Abkochung zerhacken (oder durch ein Wolf drehen) 3 bis 5 Minuten lang und 6 Stunden bis zu 10 Tagen auslaugen (mazerieren) lassen in einer Raumtemperatur oder 4°C;
e) die verflüssigte Masse im Schritt (d) filtrieren mit einem Sieb von 0.2 bis 1 mm Poren und anschliessend durch einen Papierfilter filtrieren;
f) die in Schritt (e) erhaltene Flüssigkeit in einem rotierenden Verdampfer konzentrieren bei einem niedrigen Druck und mit einer Temperatur von 35 bi zu 50°C.;
g) die konzentrierte Flüssigkeit lyaphilizieren für den Erhalt von einer lyophilizierten Abkochung mit pharmakologisch Aktivitäten.

2. Das Verfahren gemäss Ansprüche 1 charakterisiert durch das Verhältnis des Schrittes (c), dauert 5 Minuten.

3. Das Verfahren gemäss Ansprüche 1 ist charakterisiert durch das Verhältnis, dass die Zeit für das Auslaugen (Mazerierung) 24 Stunden dauert.

4. Das Verfahren gemäss Ansprüche 1 ist charakterisiert durch das Verhältnis, dass die Konzentration der Flüssigkeitsphase (Schritt f) bei 40°C erhalten wird.

5. Das Verfahren für den Erhalt von einem hydro-alkoholischen Extrakt ist charakterisiert durch das Verhältnis, dass zusätzlich zur der in Schritt (c) der Ansprüche 1 erhaltene Abkochung mit einem Ethahol-Lösungsmittel in der Proportion 1:1, v:v ausgepresst wird und währen 3 bis 5 Minuten strack verhackt wird und dann während 6 Stunden bis zu 10 Tagen ausgelaugt wird bei einer Raumtemperatur oder 4°C, für den Erhalt des hydro-alkoholischen Extrakts der Abkochung.

6. Das Verfahren gemäss Ansprüche 5 ist charakterisiert durch das Verhältnis, dass die Abkochung durch ein Sieb mit 0,2 bis 1 mm Poren gefiltert wird und anschliessend durch ein Papierfilter gefiltert wird.

7. Das Verfahren gemäss Ansprüche 6 ist charakterisiert durch das Verhältnis, dass die Flüssigkeitsphase durch einen rotierenden Verdampfer konzentriert wird bei einem niedrigen Druck und 35 bis 50°C.

8. Das Verfahren gemäss Ansprüche 7 ist charakterisiert durch das Verhältnis, dass die Flüssigkeitsphase nach der Konzentration lyophiliziert wird für den Erhalt des lyophiliziert hydro-alkoholischen Extrakts der Abkochung mit einer pharmakosdynamischer Aktivität.

9. Das Verfahren für den Erhalt eines Ethyl hydro-alkoholischen Acetats ist charakterisiert durch das Verhältnis, dass zusätzlich die erhaltene Abkochung im Schritt (c) der Ansprüche 1 ausgepresst wird mit einem Ethanol-Lösungsmittel und Ethylacetat in der Proportion 1:1:1, v:v:v und während 3 bis 5 Minuten strack verhackt wird und dann für eine Zeit von 6 Stunden bis 10 Tagen in einer Raumtemperatur oder 4°C ausgelaugt wird für den Erhalt des hydro-alkoholischen Acetats des Ethylextraktes der Abkochung.

10. Das Verfahren gemäss Ansprüche 9 ist charakterisiert durch das Verhältnis, dass die erhaltene ausgelaugte Masse mit einem Sieb von 0,2 bis 1 mm Poren gefiltert wird und dann durch Papierfilter gefiltert wird.

11. Das Verfahren gemäss Ansprüche 10 ist charakterisiert durch das Verhältnis, dass die Flüssigkeitsphase konzentriert wird in einem rotierenden Verdampfer unter einem niedrigen Druck und 35 bis 50°C.

12. Das Verfahren gemäss Ansprüche 11 ist charakterisiert durch das Verhältnis, dass die Flüssigkeitsphase nach der Konzentrierung lyophiliziert wird für den Erhalt von lyophilizierten hydro-alkoholischen Acetats Ethyl-Extraktes der Auslaugung mit einer pharmakosdynamischen Aktivität.

13. Pharmazeutische Präparate sind charakterisiert durch das Verhältnis, dass sie 200 bis 500 mg der lyophilizierten Auslaugung des *Vitis labrusca* oder *Vitis vinifera* in den Ansprüchen 1 bis 4 enthalten und zusätzlich 200 mg Kornsubtanz und 0,5 mg dioxid-Kolloidalsilikon.

14. Pharmazeutische Präparate sind charakterisiert durch das Verhältnis, dass sie 200 bis 500 mg von lyophylizierten hydro-alkoholischen Extrakt enthalten, erhalten von der Auslaugung der *Vitis labrusca* oder *Vitis vinifera* in den Ansprüchen 5 bis 8 und zusätzliche 200 mg Kornsubstanz und 0,5 mg dioxid-Kolloidalsilikon.

15. Pharmazeutische Präparate sind charakterisiert durch das Verhältnis, dass sie 200 bis 500 mg von lyophilizierten hydro-alkoholischen Acetat von Ethyl-Extrakt enthalten, erhalten von der Auslaugung des *Vitis labrusca* oder *Vitis vinifera* in den Ansprüchen 9 bis 12 und zusätzlich 200 mg Kornsubstanz und 0,5 mg dioxid-Kolloidalsilikon.

16. Die Verwendung der pharmazeutischen Präparate in den Ansprüchen 13, 14 und 15 für die Herstellung einer Medikament für die Vorbeugung und Behandlung der arteriellen Hypertonie und für die verursachten Krankheiten durch die arterielle Hypertonie.

17. Die Verwendung nach Ansprüche 16 wobei die sogennante Medikament Menschliche und Tierärztliche Anwendung hat.

## Revendications

1. Procédé pour obtenir la décoction de peau de *Vitis labrusca* ou *Vitis vinifera*, **caractérisé par** les étapes suivantes:
a) Séparez le fruit en peau et pulpe;
b) Lavez la peau obtenue à l'étape (a) en eau de robinet pendant 3 minutes;
c) Soumettez la peau de l'étape (b) à un procédé d'extraction en eau bouillante de 3 à 10 minutes pour obtenir la décoction;
d) Hachez la décoction de l'étape (c) avec un hachoir pendant une période de 3 à 5 minutes et macérez pendant une période de 6 heures à 10 jours à température ambiante ou à 4°C;
e) Filtrez le macéré obtenu à l'étape (d) dans un tamis de maille entre 0,2 et 1 mm et ensuite avec un filtre en papier;
f) Concentrez la phase liquide obtenue à l'étape (e) dans un évaporateur rotatif à basse pression et à température entre 35°C et 50°C;
g) Lyophilisez la phase liquide concentrée afin d'obtenir un lyophilisé de la décoction à activité pharmacologique;

2. Procédé, selon la revendication 1, **caractérisé en ce que** le temps de l'extraction de l'étape (c) est de 5 minutes.

3. Procédé, selon la revendication 1, **caractérisé en ce que** le temps de macération est de 24 heures.

4. Procédé, selon la revendication 1, **caractérisé en ce que** la concentration de la phase liquide (étape (f)) est obtenue à 40°C.

5. Procédé pour obtenir un extrait hydroalcoolique, **caractérisé en ce qu'**additionneliement la décoction obtenue à l'étape (c) de la revendication 1 est extraite avec le solvant éthanol à la proportion de 1:1, v:v et hachée fortement pendant une période de 3 à 5 minutes et ensuite macérée pendant une période de 6 heures à 10 jours à température ambiante ou à 4°C, obtenant, ensuite, l'extrait hydroalcoolique de la décoction.

6. Procédé, selon la revendication 5, **caractérisé en ce que** le macéré est filtré dans un tamis de maille entre 0,2 et 1 mm et ensuite avec un filtre en papier.

7. Procédé, selon la revendication 6, **caractérisé en ce que** la phase liquide est concentrée dans un évaporateur rotatif à basse pression et à température entre 35°C et 50°C.

8. Procédé, selon la revendication 7, **caractérisé en ce que** la phase liquide après être concentrée est lyophilisée pour obtenir un extrait hydroalcoolique lyophilisé de la décoction à activité pharmacodynamique.

9. Procédé pour obtenir un extrait hydroalcoolique d'acétate d'éthyle **caractérisé en ce qu'**additionnellement la décoction obtenue à l'étape (c) de la revendication 1 est extraite avec les solvants éthanol et acétate d'éthyle à la proportion de 1:1:1, v:v:v et hachée fortement pendant une période entre 3 et 5 minutes et ensuite macérée pendant une période entre 6 heures et 10 jours à température ambiante ou à 4°C, pour obtenir l'extrait hydroalcoolique d'acétate d'éthyle de la décoction.

10. Procédé, selon la revendication 9, **caractérisé en ce que** le macéré obtenu est filtré dans un tamis de maille entre 0,2 et 1 mm et ensuite avec un filtre en papier.

11. Procédé, selon la revendication 10, **caractérisé en ce que** la phase liquide est concentrée dans un évaporateur rotatif à basse pression et à température comprise entre 35°C et 50°C.

12. Procédé, selon la revendication 11, **caractérisé en ce que** la phase liquide après être concentrée est lyophilisée pour obtenir le lyophilisé de l'extrait hydroalcoolique d'acétate d'éthyle de la décoction à activité pharmacodynamique.

13. Préparations pharmaceutiques, **caractérisées en ce qu'**elles contiennent de 200 à 500 mg de lyophilisé de la décoction de *Vitis labrusca* ou *Vitis vinifera* selon l'une des revendications 1 à 4 et additionnellement 200 mg d'amide de maïs et 0,5 mg de dioxyde de silice colloïdal.

14. Préparations pharmaceutiques, **caractérisées en ce qu'**elles contiennent de 200 à 500 mg de lyophilisé de l'extrait hydroalcoolique obtenu de la décoction de *Vitis labrusca* ou *Vitis vinifera* selon l'une des revendications 5 à 8 et additionnellement 200 mg d'amide de maïs et 0,5 mg de dioxyde de silice colloïdal.

15. Préparations pharmaceutiques, **caractérisées en ce qu'**elles contiennent de 200 à 500 mg de lyophilisé de l'extrait hydroalcoolique d'acétate d'éthyle obtenu de la décoction de *Vitis labrusca* ou *Vitis vinifera* selon l'une des revendications 9 à 12 et additionnellement 200 mg d'amide de maïs et 0,5 mg de dioxyde de silice colloïdal.

16. Utilisation des préparations pharmaceutiques, selon l'une des revendications 13, 14 et 15, pour la fabrication d'un médicament pour la prévention et traitement de l'hypertension artérielle et des maladies induites par l'hypertension artérielle.

17. Utilisation, selon la revendication 16, en ce que le médicament est pour applications humaines et vétérinaires.
